# EUROPÄISCHE PATENTSCHRIFT

(11) **EP 1 360 416 B1**
(45) Veröffentlichungstag und Bekanntmachung des Hinweises auf die Patenterteilung: **19.10.2005**
(21) Anmeldenummer: 02700253.4
(22) Anmeldetag: 18.02.2002
(51) Int. Cl.: F04D 29/04, F04D 13/06, F04D 3/02, A61M 1/10

(54) **VORRICHTUNG ZUR AXIALEN FÖRDERUNG VON FLÜSSIGKEITEN**
DEVICE FOR AXIALLY CONVEYING FLUIDS
DISPOSITIF DE TRANSPORT AXIAL DE LIQUIDES

(30) Priorität: 16.02.2001 DE 10108810
(43) Veröffentlichungstag der Anmeldung: 12.11.2003
(73) Patentinhaber: Berlin Heart AG, 12247 Berlin (DE)
(72) Erfinder: NÜSSER, Peter, 13051 Berlin (DE); MÜLLER, Johannes, 10717 Berlin (DE); PETERS, Hans-Erhard, 10437 Berlin (DE); MÜLLER, Jörg, 10439 Berlin (DE); NEUMANN, Werner, 12247 Berlin (DE); GRAICHEN, Kurt, 13189 Berlin (DE); ARNDT, Andreas, 12489 Berlin (DE)
(74) Vertreter: Golkowsky, Stefan
(86) Internationale Anmeldenummer: PCT/EP2002/001740
(87) Internationale Veröffentlichungsnummer: WO 2002/066837

(56) Entgegenhaltungen:
- WO-A-00/64508
- WO-A-98/11650

## Beschreibung

Die Erfindung betrifft eine Vorrichtung zur axialen Förderung von Flüssigkeiten gemäß dem Oberbegriff des Anspruches 1.

Insbesondere geringer stabile mehrphasige Fluide, die durch einen Energieeintrag irreversible Veränderungen erfahren können, wie z. B. Emulsionen und Dispersionen, können beim Fördern in entsprechenden Vorrichtungen wie Pumpen nachteiligerweise in instabile Bereiche geraten.

Ein besonders empfindliches Fluidsystem stellt das Blut dar. Diese undurchsichtige rote Körperflüssigkeit der Wirbeltiere zirkuliert in einem in sich geschlossenen Gefäßsystem, wobei rhythmische Kontraktionen des Herzens das Blut in die verschiedenen Gebiete des Organismus hineindrücken. Hierbei transportiert das Blut die Atemgase Sauerstoff und Kohlendioxid sowie Nährstoffe, Stoffwechselprodukte und körpereigene Wirkstoffe. Das Blutgefäßsystem einschließlich des Herzens ist hierbei hermetisch von der Umwelt abgeschirmt, so dass im gesunden Organismus das Blut vom Stoffaustausch mit den Körperzellen abgesehen keine Veränderungen erfährt, wenn es über das Herz durch den Körper gepumpt wird.

Bekannt ist, dass das Blut bei Kontaktierung mit nichtkörpereigenen Materialien oder durch Fremdenergieeinwirkung zur Hämolyse und Thrombenbildung neigt. Thrombenbildung kann für den Organismus tödlich sein, weil sie zu Verstopfungen im weitverzweigten Gefäßsystem führen kann. Hämolyse beschreibt den Zustand, dass über das physiologische Maß hinaus die roten Blutkörperchen innerhalb des Körpers lysiert - zerstört - werden. Die Ursachen für Hämolyse können mechanisch oder metabolischer Art sein. Gesteigerte Hämolyse hat multiple Organschäden zur Folge und kann bis zum Tode des Menschen führen.

Andererseits hat sich gezeigt, dass es prinzipiell möglich ist, unter bestimmten konstruktiven Voraussetzungen die Pumpleistung des Herzens zu unterstützen bzw. sogar das natürliche Herz durch ein Kunstherz zu ersetzen. Allerdings ist ein Dauerbetrieb von implantierten Herzunterstützungspumpen oder Kunstherzen zur Zeit nur begrenzt möglich, weil die Wechselwirkungen dieser Kunstprodukte mit dem Blut und dem gesamten Organismus immer noch zu nachteiligen Veränderungen des Blutes und des Organismus führen.

Aus dem Stand der Technik sind axiale Blutpumpen bekannt, die im wesentlichen aus einem zylindrischen Rohr bestehen, in dem ein Förderteil, das als Rotor eines außen anliegenden Motorstators ausgebildet ist, rotiert. Der Rotor, der eine sogenannte Beschaufelung aufweist, fördert, nachdem er in Rotation versetzt wurde, die Flüssigkeit in axialer Richtung. Die Lagerung dieser sogenannten Axialpumpen stellt ein großes Problem dar. Eine rein mechanische Lagerung ist hinsichtlich'der Blutschädigung und auch der relativ hohen Reibungswerte nachteilig. Auch die bisher beschriebenen Magnetlagerungsvarianten haben insbesondere zu keiner befriedigenden Lösung für die Lagerungsverhältnisse in Axialpumpen geführt, bei denen eine sehr kompakte Bauweise angestrebt wird.

So wird in der WO 00/64030 eine Vorrichtung zur schonenden Förderung von ein- oder mehrphasigen Fluiden beschrieben, deren Förderteil ausschließlich magnetisch gelagert ist. Hierzu sind in das Förderteil bevorzugt sowohl permanentmagnetische Lagerelemente für die Magnetlagerung als auch permanentmagnetische Elemente für die Funktionalität als Motorrotor eines Elektromotors integriert. Die Verwendung einer Magnetlagerung für die hier beschriebene Fördereinrichtung erlaubt es, auf üblicherweise in der Strömung des zu fördernden Fluides angeordnete Lagerelemente, die zu Totwassergebieten und Verwirbelungen des zu fördernden Fluides führen und dadurch die Strömung in negativer Weise beeinflussen, zu verzichten.
Die hier beschriebene magnetische Lagerung nimmt sowohl die axialen als auch die-radialen Kräfte auf. Die axiale Lage des Förderteiles wird aktiv stabilisiert, während die radiale Lagerung des Förderteiles mittels der vorhandenen Permanentmagnete ausschließlich passiv erfolgt. Die beschriebene Fördereinrichtung weist allerdings mehrere Nachteile auf.

Die passive magnetische Radiallagerung ist durch eine relativ geringe radiale Steifigkeit und Dämpfung charakterisiert, wodurch beim Pumpvorgang Probleme beim Durchfahren kritischer Drehzahlen des Rotors bzw. des Lagers auftreten. Eventuell vorhandene hydrodynamische und mechanische Unwuchten des Rotors haben gravierende Auswirkungen auf die Funktion der Pumpe, insbesondere in der Anwendung als blutfördernde Einrichtung.

Die WO 98/11650 zeigt eine Rotationspumpe sowie ein Verfahren zum Betrieb derselben. So zeigt die D1 eine Vorrichtung zur axialen Förderung von Flüssigkeiten, bestehend aus einem rohrförmigen, die Flüssigkeit im wesentlichen axial führenden Hohlkörper, in dem in axialer Ausrichtung ein mit einem außerhalb des Hohlkörpers befindlichen Motorstator in Rotation versetzbares magnetisch gelagertes Förderteil angeordnet ist. Dieses Förderteil weist beispielsweise in Figur 5a der D1 gezeigten Ausführungsform einen gegenüber der Abmessung in axialer Richtung sehr großen Durchmesser auf. Zur Stabilisierung ist daher u.a. ein hydrodynamisches Axiallager vorgesehen, das durch einen Außenkranz des Rotors geführt ist. Zusätzlich ist hier auch eine hydrodynamische Radiallagerung möglich, welche u.U. eine aktive magnetische Radiallagerung unterstützt.

Der Erfindung liegt die Aufgabe zugrunde, eine Vorrichtung zur axialen Förderung von Flüssigkeiten anzubieten, deren Förderteil vollständig magnetisch gelagert ist und dessen radiale Lagerung eine hinreichende Steifigkeit und eine wirkungsvolle Dämpfung aufweist, so dass Probleme beim Durchfahren kritischer Drehzahlen sowie nachteilige Auswirkungen hydrodynamischer und mechanischer Unwuchten des Rotors vermieden werden können.

Die Lösung der Aufgabe erfolgt mit einer Vorrichtung zur axialen Förderung von Flüssigkeiten gemäß Anspruch 1.

So weist die Vorrichtung zur axialen Förderung von flüssigkeiten, bestehend aus einem rohrförmigen, die Flüssigkeit im wesentlichen axial führenden Hohlkörper, in dem in axialer Ausrichtung ein mit einem außerhalb des Hohlkörpers befindlichen Motorstator in Rotation versetzbares magnetisch gelagertes Förderteil angeordnet ist, wobei das eine magnetische Lagerung aufweisende Förderteil eine Rotorbeschaufelung aufweist, eine mit einer hydrodynamischen Lagerung kombinierte Lagerung auf. Die Lagerung des Förderteiles ist eine aktiv stabilisierte magnetische Axiallagerung, eine passive magnetische Radiallagerung und eine hydrodynamische Radiallagerung.

Vorteilhafte Weiterbildungen sind in den Unteransprüchen angegeben.

Die hydrodynamische Radiallagerung ist in einer weiteren Ausbildung der Erfindung als hohlzylindrischer, rotationssymmetrischer Stützring, der mit dem Förderteil verbunden ist, ausgebildet.

Auf dem Förderteil ist mindestens ein Stützring angeordnet, wobei die Stützringe am Anfang des Motorrotors und/oder am Ende des Motorrotors oder zwischen diesen genannten Positionen angeordnet sind.

In einer Weiterbildung der Erfindung entspricht die axiale Abmessung des Stützringes maximal der axialen Länge des Förderteiles, und die axiale Abmessung der Lauffläche des Stützringes ist kleiner als eine Innenfläche des Stützringes.

Der Stützring weist die gleiche radiale Abmessung auf wie die Rotorbeschaufelung und ist mit ihr verbunden.

Weiterbildend besitzt der Stützring eine solche radiale Abmessung (Dicke), dass er mit einer radialen Profilierung versehen werden kann, die der Konditionierung der Zuströmung in die Rotorbeschaufelung des Förderteils dient.

In einer weiteren Ausbildung ist ein Stützring mit derartiger axialer Erstreckung vorgesehen, dass die Beschaufelung auf ihrer gesamten Länge radial vom Stützring begrenzt wird. Die Lauffläche des Stützringes, die gegen die Innenseite des rohrförmigen Hohlkörpers zeigt, weist vorteilhafterweise eine Oberflächenbeschichtung mit Notlaufeigenschaften auf, die zudem biokompatibel ist.

Die Innenfläche des Stützringes hat in einer Ausführung eine Profilierung, die die Strömungseigenschaften günstig beeinflussen kann.

Die Ausbildung der Lauffläche des Stützringes als eine Lauflinie führt zu besonders.günstigen Reibungswerten.

Die große Steifigkeit und Dämpfung der radialen Lagerung des Förderteils wird dadurch erreicht, dass zusätzlich zu einer magnetischen Lagerung des Förderteiles eine hydrodynamische Lagerung vorgesehen ist. Die hydrodynamische Lagerung wird durch mindestens einen hohlzylindrischen, rotationssymmetrischen Stützring erreicht, der mit dem Förderteil fest verbunden ist. Bei geeigneter Ausbildung des Stützringes wird dem Rotor eine große Kippsteifigkeit verliehen. Vorteilhafterweise wird dieser Effekt durch eine besonders große axiale Erstreckung des Stützringes oder durch die Anbringung mindestens zweier Stützringe an einem Rotor erreicht.

Bei großer axialer Erstreckung des Stützringes bzw. weitgehender oder vollständiger Kapselung der Beschaufelung durch einen solchen Stützring, werden vorteilhafterweise schädliche Wirkungen des an den Schaufelenden auftretenden radialen Spaltes vermieden.

Die Erfindung wird anhand einer Zeichnung näher erläutert:

Es zeigen
- Fig. 1: eine schematische Darstellung eines Axialschnittes einer axialen Blutpumpe mit Stützring,
- Fig. 2: eine schematische Darstellung einer Anordnung einer Anordnung eines Stützringes auf dem Rotor,
- Fig. 3: eine schematische Darstellung einer Anordnung zweier Stützringe auf dem Rotor.
- Fig. 4: eine schematische Darstellung einer Anordnung eines Stützringes mit profitierter Innenfläche,
- Fig. 5: eine schematische Darstellung eines über den gesamten Rotors sich erstreckenden Stützringes und
- Fig. 6: eine schematische Darstellung eines Stützringes auf dem Rotor mit einer Lauflinie auf der Lauffläche.

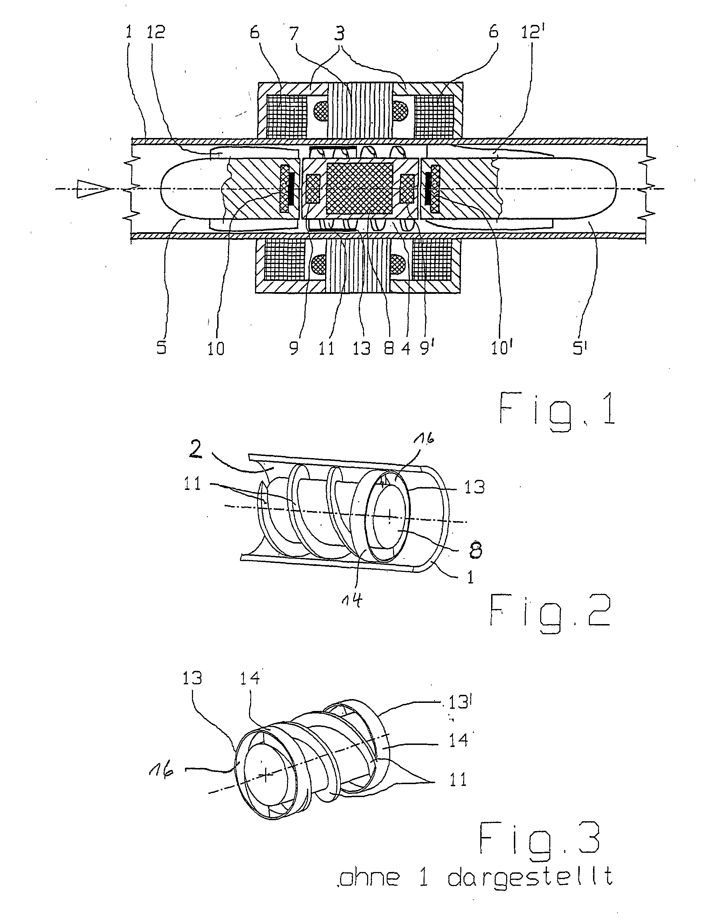

Fig. 1 zeigt beispielhaft in axialer Schnittdarstellung den Aufbau einer gattungsmäßigen Axialpumpe mit der erfindungsgemäßen Lagerung eines Förderteiles 4. Die Axialpumpe besteht in ihren Hauptteilen aus einem rohrförmigen Hohlkörper 1 und einem Pumpengehäuse 3, das einen Motorstator 7 und Axialstabilisatoren 6 einschließt. Das Pumpengehäuse 3 liegt unmittelbar rotationssymmetrisch am rohrförmigen Hohlkörper 1 an. Im Inneren des rohrförmigen Hohlkörpers 1 sind eine Fluidvorleiteinrichtung 5 und eine Fluidnachleiteinrichtung 5' vorgesehen, zwischen denen das mittels des Motorstators 7 in Rotation versetzbare Förderteil 4 angeordnet ist.

Das Förderteil 4 ist magnetisch gelagert, wobei permanent magnetische Lagerelemente 9 und 9' im Motorrotor 8 und permanentmagnetische Lagerelemente 10 und 10' in den Fluidvor- und Fluidnachleiteinrichtungen 5 und 5' angeordnet sind. Auf dem Motorrotor 8 des Förderteiles 4 ist eine Rotorbeschaufelung 11 vorgesehen, die mit einem Stützring 11 kombiniert ist. Das magnetisch gelagerte Förderteil 4 wird über den Motorstator 7 in Rotation versetzt, wobei mittels der sich gegenüberstehenden permanentmagnetischen Lagerelemente 9, 9' und 10, 10' in Verbindung mit den Axialstabilisatoren 6 das Förderteil schwebend gehalten wird und der Stützring 11 für eine zusätzliche hydrodynamische Lagerung des rotierenden Förderteiles 4 sorgt.

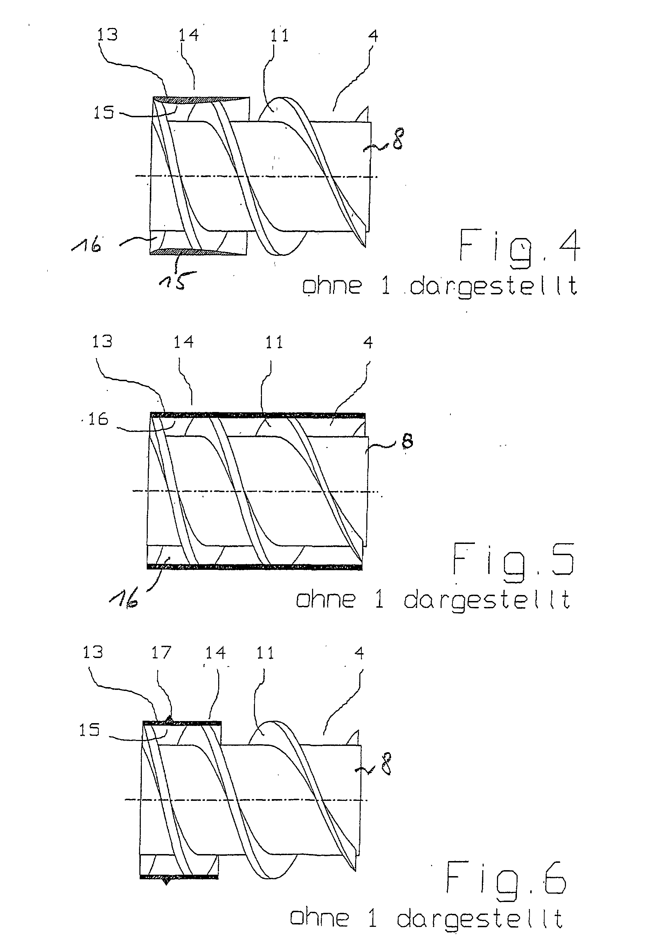

Fig. 2 zeigt in schematischer Darstellung den Motorrotor 8 mit der Rotorbeschaufelung 11 in einem aufgeschnittenen rohrförmigen Hohlkörper 1. Erfindungsgemäß ist der Stützring 13 hier im Endbereich des Motorstators 8 angeordnet. Die zu fördernde Flüssigkeit wird hier zwischen einer Innenfläche 16 des Stützringes 13 und dem Motorrotor 8 bewegt. Eine Lauffläche 14 des Stützringes 13 wird bei minimalem Abstand zu einer Innenwandung 2 des rohrförmigen Hohlkörpers 1 bewegt.

Fig. 3 zeigt in schematischer Darstellung die Anordnung von zwei Stützringen 13 und 13' an den Enden eines Motorrotors 8. Die Darstellung des rohrförmigen Hohlkörpers 1 ist hier weggelassen.

Fig. 4 zeigt eine weitere erfindungsgemäße Ausgestaltung des Stützringes 13. Die Innenfläche 16 des Stützringes 13 zeigt eine Profilierung 15. Wie in der Schnittdarstellung des Stützringes 13 erkennbar ist hier die Profilierung 15 in tragflächenähnlicher Form ausgeführt. Auch hier ist auf die Darstellung des rohrförmigen Hohlkörpers verzichtet worden.

In einer weiteren Weiterbildung der Erfindung ist in Fig. 5, ebenfalls ohne Darstellung des rohrförmigen Hohlkörpers 1 ein Stützring 13 angeordnet, der die gesamte axiale Länge des Motorrotors 8 mit seiner Beschaufelung 11 befaßt. Die Beförderung der Flüssigkeit erfolgt auch hier zwischen der Innenfläche 16 des Stützringes 13 und dem Motorrotor 8.

In einer weiteren Ausgestaltung der Erfindung ist in Fig. 6 ein Stützring 13 dargestellt, der seiner Lauffläche 14 eine erhabene Lauflinie 17 aufweist, die einen minimalen Abstand verbunden mit einer minimalen Reibung gegenüber der Innenwandung 2 des rohrförmigen Hohlkörpers 1 ermöglicht.

### Bezugszeichenliste

- 1: rohrförmiger Hohlkörper
- 2: Innenwandung
- 3: Pumpengehäuse
- 4: Förderteil
- 5: Fluidvorleiteinrichtung
- 5': Fluidnachleiteinrichtung
- 6: Axialstabilisator
- 7: Motorstator
- 8: Motorrotor
- 9: permanentmagnetisches Lagerelement
- 9': permanentmagnetisches Lagerelement
- 10: permanentmagnetisches Lagerelement
- 10': permanentmagnetisches Lagerelement
- 11: Rotorbeschaufelung
- 12: Fluidleitbeschaufelung
- 12': Fluidleitbeschaufelung
- 13: Stützring
- 13': Stützring
- 14: Lauffläche
- 15: Profilierung
- 16: Innenfläche
- 17: Lauflinie

## Patentansprüche

1. Vorrichtung zur axialen Förderung von Flüssigkeiten, bestehend aus einem rohrförmigen, die Flüssigkeit im Wesentlichen axial führenden Hohlkörper (1), in dem in axialer Ausrichtung ein mit einem außerhalb des Hohlkörpers (1) befindlichen Motorstator (7) in Rotation versetzbares magnetisch gelagertes Förderteil (4) angeordnet ist, wobei das eine magnetische Lagerung aufweisende Förderteil (4) eine Rotorbeschaufelung (11) aufweist, und die magnetische Lagerung mit einer hydrodynamischen Radiallagerung kommoiniert ist, **dadurch gekennzeichnet, dass** die magnetische Lagerung des Förderteils als eine aktiv stabilisierte magnetische Axiallagerung und eine passive magnetische Radiallagerung ausgeführt ist.

2. Vorrichtung- nach Anspruch 1,
**dadurch gekennzeichnet, dass**
die hydrodynamische Radiallagerung als hohlzylindrischer rotationssymmetrischer Stützring (13), der mit dem Förderteil (4) verbunden ist, ausgebildet ist.

3. Vorrichtung nach Anspruch 1 oder 2,
**dadurch gekennzeichnet, dass**
auf dem Förderteil (4) mindestens ein Stützring (13) angeordnet ist.

4. Vorrichtung nach einem der Ansprüche 1 bis 3,
**dadurch gekennzeichnet, dass**
die Stützringe (13) am Anfang des Motorrotors (8) und/oder am Ende des Motorrotors (8) oder zwischen diesen genannten Positionen angeordnet sind.

5. Vorrichtung nach einem der Ansprüche 1 bis 4,
**dadurch gekennzeichnet, dass**
die axiale Abmessung des Stützringes (13) maximal der axialen Länge des Förderteiles (4) entspricht.

6. Vorrichtung nach einem der Ansprüche 1 bis 5,
**dadurch gekennzeichnet, dass**
die axiale Abmessung der Lauffläche (14) des Stützringes (13) kleiner als eine Innenfläche (16) des Stützringes (13) ist.

7. Vorrichtung nach einem der Ansprüche 1 bis 6 ,
**dadurch gekennzeichnet, dass**
der Stützring (13) die gleiche radiale Abmessung aufweist wie die Rotorbeschaufelung (11).

8. Vorrichtung- nach einem der Ansprüche 1 bis 7,
**dadurch gekennzeichnet, dass**
Stützring (13) und Rotorbeschaufelung (11) verbunden sind.

9. Vorrichtung nach einem der Ansprüche 1 bis 8,
**dadurch gekennzeichnet, dass**
der Stützring (13) eine solche radiale Abmessung (Dicke) besitzt, so dass er mit einer radialen Profilierung versehen werden kann, die der Konditionierung der Zuströmung in die Rotorbeschaufelung (11) des Förderteils (4) dient.

10. Vorrichtung nach einem der Ansprüche 1 bis 9,
**dadurch gekennzeichnet, dass**
ein Stützring (13) mit derartiger axialer Erstreckung vorliegt, dass die Beschaufelung (11) auf ihrer gesamten Länge radial vom Stützring (13) begrenzt wird.

11. Vorrichtung nach einem der Ansprüche 1 bis 10,
**dadurch gekennzeichnet, dass**
die Lauffläche (14) des Stützringes (13), die gegen die Innenseite des rohrförmigen Hohlkörpers (1) zeigt, eine Oberflächenbeschichtung mit Notlaufeigenschaften aufweist, die zudem biokompatibel ist.

12. Vorrichtung nach einem der Ansprüche 1 bis 11,
**dadurch gekennzeichnet, dass**
die Innenfläche (16) des Stützringes (13) eine Profilierung (15) aufweist.

13. Vorrichtung nach einem der Ansprüche 1 bis 12,
**dadurch gekennzeichnet, dass**
die Lauffläche (14) des Stützringes (13) eine Lauflinie (17) aufweist.

## Claims

1. Device for axial conveyance of fluids, comprising a tubular hollow body (1) which essentially ducts a fluid axially, wherein is arranged in axial alignment a magnetically mounted conveying part (4) which is rotated by a motor stator (7) located outside the hollow body (1), and the magnetically mounted conveying part (4) comprises a rotor blading (11), and the magnetic mounting is combined with a hydro-dynamic radial mounting, **characterised in that** the magnetic mounting of the conveying part of the conveying part is designed as an actively stabilised magnetic axial mounting and a passive magnetic radial mounting.

2. Device according to Claim 1, **characterised in that** the hydro-dynamic radial mounting is designed as a hollow cylindrical, rotation-symmetric support ring (13) which is connected to the conveying part (4).

3. Device according to Claim 1 or 2, **characterised in that** at least one support ring (13) is arranged on the conveying part (4).

4. Device according to one of Claims 1 to 3, **characterised in that** the support rings (13) are arranged at the beginning of the motor rotor (8) and/or at the end of the motor rotor (8) or between these positions.

5. Device according to one of Claims 1 to 4, **characterised in that** the axial dimension of the support ring (13) corresponds maximally with the axial length of the conveying part (4).

6. Device according to one of Claims- 1 to 5, **characterised in that** the axial dimension of the running surface (14) of the support ring (13) is smaller than an inside surface (16) of the support ring (13).

7. Device according to one of Claims 1 to 6, **characterised in that** the support ring (13) has the same radial dimension as the rotor blading (11).

8. Device according to one of Claims 1 to 7, **characterised in that** the support ring (13) and the rotor blading (11) are linked.

9. Device according to one of Claims 1 to 8, **characterised in that** the support ring (13) has a radial dimension (thickness) which allows it to be provided with a radial profile which serves to condition the inflow into the rotor blading (11) of the conveying part (4).

10. Device according to one of Claims 1 to 9, **characterised in that** a support ring (13) has an axial extent which radially defines the blading (11) over its entire length from the support ring (13).

11. Device according to one of Claims 1 to 10, **characterised in that** the running surface (14) of the support ring (13) which points against the inside of the tubular hollow body (1) comprises a surface coating with emergency running properties which is also bio-compatible.

12. Device according to one of Claims 1 to 11, **characterised in that** the inside surface (16) of the support ring (13) comprises a profile (15).

13. Device according to one of Claims 1 to 12, **characterised in that** the running surface (14) of the support ring (13) comprises a running line (17).

## Revendications

1. Dispositif de refoulement axial de fluides, constitué d'un corps creux (1) tubulaire, guidant le fluide sensiblement axialement, dans lequel est disposé, dans la direction axiale, un élément de refoulement (4) supporté magnétiquement, apte à être mis en rotation avec un stator de moteur (7) se trouvant à l'extérieur du corps creux (1), l'élément de refoulement (4) supporté magnétiquement comportant un aubage de rotor (11), et le supportage magnétique étant combiné à un supportage radial hydrodynamique, **caractérisé en ce que** le supportage magnétique de l'élément de refoulement est réalisé sous la forme d'un supportage magnétique axial stabilisé actif, et d'un supportage magnétique radial passif.

2. Dispositif selon la revendication 1, **caractérisé en ce que** le supportage radial hydrodynamique est configuré sous la forme d'un anneau de support (13), symétrique en rotation, et de type cylindre creux, qui est relié à l'élément de refoulement (4).

3. Dispositif selon l'une quelconque des revendications 1 ou 2, **caractérisé en ce que**, sur l'élément de refoulement (4), est disposé au moins un anneau de support (13).

4. Dispositif selon l'une quelconque des revendications 1 à 3, **caractérisé en ce que** les anneaux de support (13) sont disposés à l'origine du rotor du moteur (8) et / ou à l'extrémité du rotor du moteur (8), ou entre ces positions mentionnées.

5. Dispositif selon l'une quelconque des revendications 1 à 4, **caractérisé en ce que** la dimension axiale de l'anneau de support (13) correspond au maximum à la longueur axiale de l'élément de refoulement (4).

6. Dispositif selon l'une quelconque des revendications 1 à 5, **caractérisé en ce que** la dimension axiale de la surface de roulement (14) de l'anneau de support (13) est inférieure à une surface intérieure (16) de l'anneau de support (13).

7. Dispositif selon l'une quelconque des revendications 1 à 6, **caractérisé en ce que** l'anneau de support (13) présente la même dimension radiale que l'aubage du rotor (11).

8. Dispositif selon l'une quelconque des revendications 1 à 7, **caractérisé en ce que** l'anneau de support (13) et l'aubage du rotor (11) sont reliés l'un à l'autre.

9. Dispositif selon l'une quelconque des revendications 1 à 8, **caractérisé en ce que** l'anneau de support (13) a une étendue radiale (épaisseur) telle qu'il peut être doté d'un profilage radial, qui sert à conditionner l'affluence dans l'aubage du rotor (11) de l'élément de refoulement (4).

10. Dispositif selon l'une quelconque des revendications 1 à 9, **caractérisé en ce qu'**un anneau de support (13) a une étendue axiale telle que l'aubage (11) est délimité radialement sur toute sa longueur par l'anneau de support (13).

11. Dispositif selon l'une quelconque des revendications 1 à 10, **caractérisé en ce que** la surface de roulement (14) de l'anneau de support (13), qui est orienté face au côté intérieur du corps creux (1) tubulaire, comporte un revêtement superficiel ayant des propriétés de fonctionnement en cas d'urgence, et, en outre, étant biocompatible.

12. Dispositif selon l'une quelconque des revendications 1 à 11, **caractérisé en ce que** la surface intérieure (16) de l'anneau de support (13) présente un profilage (15).

13. Dispositif selon l'une quelconque des revendications 1 à 12, **caractérisé en ce que** la surface de roulement (14) de l'anneau de support (13) présente une ligne de roulement (17).
